# EUROPEAN PATENT SPECIFICATION

(11) **EP 3 977 489 B1**
(45) Date of publication and mention of the grant of the patent: **24.04.2024**
(21) Application number: 20728062.9
(22) Date of filing: 28.05.2020
(51) Int. Cl.: A61B 5/1455, H01B 7/08

(54) **APPARATUS FOR MEASURING OPTICAL PARAMETERS IN SCATTERING MEDIA OFFERING INCREASED LIFETIME**
VORRICHTUNG ZUR MESSUNG OPTISCHER PARAMETER IN STREUENDEN MEDIEN MIT ERHÖHTER LEBENSDAUER
APPAREIL POUR MESURER DES PARAMÈTRES OPTIQUES DANS DES MILIEUX DISPERSIFS OFFRANT UNE DURÉE DE VIE ACCRUE

(30) Priority: 28.05.2019 EP 19177066
(43) Date of publication of application: 06.04.2022
(73) Proprietor: Universität Zürich, 8006 Zürich (CH)
(72) Inventor: KLEISER, Stefan, 79639 Grenzach-Wyhlen (DE); OSTOJIC, Daniel, 8046 Zürich (CH); WOLF, Martin, 8038 Zürich (CH)
(74) Representative: Mertzlufft-Paufler, Cornelius
(86) International application number: PCT/EP2020/064863
(87) International publication number: WO 2020/239921

(56) References cited:
- US-A- 5 073 683
- US-A1- 2010 210 924
- US-A1- 2014 371 548
- US-A1- 2019 000 317

## Description

### Summary of the invention

The invention is set out in the appended set of claims.

### Summary of the disclosure

The disclosure concerns an elongated and bendable apparatus for measuring at least one optical or physiological parameter in a scattering medium. This apparatus comprises at least one light source and at least one detector arranged on a carrier substrate at a distal end of said apparatus such that said at least one detector can detect at least one wavelength, emitted by said at least one light source and scattered by said medium, an electronic unit configured to control said at least one light source and to evaluate electrical signals from said at least one detector for computing said parameter and located at a proximal end of said apparatus, and an interconnection connecting said electronic unit with said carrier substrate via several tracks running in a longitudinal axis of said apparatus. When the interconnection is fully elongated and flattened, said tracks may define a first plane.

Such apparatuses are already employed in clinical use as near-infrared spectroscopy (NIRS)-oximeters for measuring cerebral oxygenation, in particular for vital-parameter-monitoring of infants. For this purpose, a sensor head of the apparatus, which bears the bendable carrier substrate with the light sources and detectors, is bent around the head of the infant to achieve good skin contact, which is crucial for a reliable measurement.

Although such apparatuses offer flexibility to be bent and applied to the highly curved surface of the small heads of infants, it has been found that a typical failure mechanism in clinical use is the formation of cracks and ruptures, in particular in the area of the electrical interconnection, due to the countless mechanical bending during use. This failure mechanism is critical, because it can lead to total failure of the apparatus, a scenario that is to be avoided as it poses a severe threat to the life of the infant, whose vital parameters are monitored with the apparatus.

It is therefore a primary object of the present invention to improve the robustness of the apparatus against such failure mechanisms and to increase its lifetime accordingly. Furthermore, it is an object of the invention to mitigate the consequences of said failure mechanism and - in the best case - to avoid total failure of the apparatus.

Relevant prior art can be found in patent documents US 2014/371548 A1, US 2010/210924 A1 and US 5 073 683 A.

In accordance with a first aspect of the present disclosure an apparatus is provided which achieves these objectives. In particular, the disclosure proposes an apparatus as introduced at the beginning, which, in addition, is characterized in that said apparatus features a bendable strain relief device. This strain relief device is configured to transmit tensile stress from the electronic unit to the carrier substrate along said longitudinal axis. In other words, the strain relief device can take up and deliver tensile forces applied between the carrier substrate and the electronic unit. For transmitting said tensile stress, the strain relief device offers two tensile stress lines, which are spaced from each other.

When the stress relief device is fully elongated and flattened, these two tensile stress lines may define a second plane oriented substantially parallel to the first plane, which was mentioned to be defined by the tracks of the interconnection.

In other words, it is suggested to provide stress relief through a purely mechanical stress relief device as a separate component. The stress relief device is provided in the form of two tension threads. Most preferably, this tension thread may have the shape of a flat band or ribbon.

As already explained, the stress relief device offers at least two tensile stress lines for transmitting stress from a distal end of the apparatus to its proximal end and vice versa. Ideally, the stress lines should be spaced at least 5 mm from each other such that torque applied in the plane of the stress relief device can be taken up at least to some extent as well. A stress line may be formed for example by a continuous thread of material of the stress relief device, in particular by a separate tension cord, such that a stress applied at one end of the device is deliverable to its other end.

Any stress applied along the longitudinal axis of the apparatus between the proximal and distal end of the stress relief device will thus be taken up by two tensile stress lines. The stress relief device thus protects the delicate electrical structures of the apparatus, in particular the tracks of the interconnection which deliver electrical control and drive signals from the electronic unit to the light sources and electrical read-outs from the detectors to the electronic unit, from excessive strain and resulting ruptures and electrical failures. Thus the strain relief device at first sight serves a purely mechanical function but also greatly enhances the electrical reliability of the apparatus in typical practical use cases.

A stress relief device according to the disclosure should therefore be resilient to tensile forces but not necessarily to compressive forces, i.e. it may be bendable and thus compressible but should show minimal stretching when put under tensile forces.

The electronic unit may feature a mechanical and/or electrical and/or optical coupling element, in particular in the form of a flat cable plug, for connecting said interconnection to said electronic unit. From this coupling element, electrical and/or optical tracks may run to electronic and/or optical components of said electronic unit. In addition, part of the electronic unit may be provided by an external mobile device such as a tablet.

The interconnection may be an electrical interconnection in which case said tracks may be conductive tracks. Via such conductive tracks control signals and/or measurement signals may be exchanged between the electronic unit and the carrier substrate, which may be designed as a measuring printed circuit board. In this case, the light sources and detectors arranged on the carrier substrate / measuring printed circuit board may be electronic devices such as light emitting devices (LEDs) or photodiodes, respectively, for example.

Alternatively, the interconnection may be an optical interconnection in which case said tracks may be in the form of optical fibers. Via such fibers, measurement light produced by LEDs located in the electronic unit may be sent to a distal fiber end facet located at the carrier substrate and serving as a light source. Likewise, such distal end facets of the optical fibers or other optical light receiving surfaces may be used at the location of the carrier substrate as detectors.

In other words, the optical fibers of the optical interconnection may be used for sending measurement light received by such optical detectors via the fibers to the electronic unit, where the received light may be electronically detected by a photodiode or the like. In the extreme case, the carrier substrate may bear no electronic components at all but only light sources and detectors formed by purely optical components, respectively.

In all of the above cases, it is of great advantage for reliable optical measurements, if the carrier board, in particular said measuring printed circuit board, defines the individual distances between the light sources and detectors of the apparatus. Theses distances, which are normally denoted as source-detector-separations (SDS) are important measurement parameters that have to be accurately controlled, in particular if said carrier board is bendable, which is necessary, for example, in clinical NIRS applications.

Thus, the carrier substrate may be bendable, in particular such that the carrier substrate can be bent onto a curved surface of said medium during a measurement, for example onto the head of a neonate. This may be achieved by using a flexible material or by combining stiff elements with flexible elements, for example by solid joints.

Furthermore, the interconnection may feature mechanical tracks, for example fibers from Aramid or Kevlar, for reinforcement.

According to a preferred embodiment, the interconnection can be designed as a semi-flexible printed circuit board (PCB) based on polyimid films and copper conductive tracks and using FR4 as the material for local reinforcements for partly stiffening said PCB.

Preferably, such stiffening can be optimized by adding reinforcements locally on both sides of the flexible PCB.

The apparatus according to the disclosure thus offers a flexible strain relief device configured to take up stress loads which are applied to the apparatus, in particular when longitudinal stress is applied to the apparatus at its distal and proximal ends. This may be the case, for example, if a cable attached to the proximal end of the apparatus is suddenly pulled accidentally.

Due to the two spaced tensile stress lines, the strain relief device cannot only take up longitudinal stress loads but also moments of force in said second plane. For example, said strain relief device may be formed as a ribbon with a width of at least 10 mm. In this case, the strain relief device offers a lever arm of ≥ 10 mm for taking up moments of force.

In other words, due to the two stress lines, the strain relief device is configured to take up a torque applied around an axis, which is perpendicular to the longitudinal axis. In yet other words, the strain device can be described as configured to take up shear forces applied in said first plane. These features are of major advantage for avoiding ruptures and cracks typically occurring at the points at which the interconnection is mechanically linked to the measuring printed board and the electronic unit, respectively, and which occur, in particular, if shear forces are applied due to twisting and bending of the apparatus during use.

At the same time, due to the parallel orientation of the two stress lines with respect to the first plane defined by the tracks, the strain relief device does not restrict out of plane bending of the interconnection, such that the desired flexibility of the apparatus, which is critical for achieving a good skin contact and hence reliable measurements, can be maintained. In other words, the strain relief is configured to be bent together with said interconnection out of said first plane.

As a result, the apparatus can take up much higher mechanical loads, in particular longitudinal stress loads as well as loads resulting from shear forces, because the strain relief device reliefs strain produced by mechanical stress from the interconnection. In effect, ruptures and cracks occur much less frequently as compared to previous designs without such a strain relief device.

The two tensile stress lines, are be anchored to the electronic unit by two proximal anchor points spaced from each other and to said measuring printed circuit by two distal anchor points spaced from each other.

A large spacing between the two stress lines is tantamount to a large lever arm for taking up torque. Therefore, it is preferred if the proximal anchor points and/or said distal anchor points lie outside of a longitudinal projection of said tracks, because this increases the torque that the strain relief device can take up. For this purpose, the carrier substrate and the electronic unit can be each wider in the first plane than a mid-section of the interconnection. This mid-section may link a distal part of the apparatus with a proximal part of the apparatus. In this case, the anchor points may each lie outside of a longitudinal projection of said mid-section of said interconnection.

The strain relief device may be fabricated along with said interconnection, in particular as an integral part of said interconnection, or separately from said interconnection. In the latter case, the device may be fixed, preferably glued, to said interconnection.

To provide sufficient strength against stress loads, the strain relief device should feature a cross-section A and be fabricated from a material with a Young's modulus E such that a product of A times E is greater than 2 kN. Concerning suitable materials for the device, it is preferable to choose a material such that the Young's modulus E is greater than 20 GPa. Suitable materials are in particular fibre-reinforced composites, because they offer a high Young's modulus as well as sufficient flexibility.

The single electronic components of the electronic unit may be mounted on a, preferably stiff, evaluation printed circuit board. In this case, the strain relief device may be mechanically connected to the evaluation printed circuit board of the electronic unit and/or to said carrier substrate by glueing or screws or rivets or like means, respectively. For such connections, it is preferable for a robust construction if a connecting device - for example a screw or a rivet - delivering a connection of said strain relief device to the evaluation printed circuit board or the carrier substrate, respectively, perforates said evaluation printed circuit board or carrier substrate.

According to a preferred embodiment, the interconnection may be designed as a flat cable, preferably with an aspect ratio (AR) smaller than 1:3 (AR = thickness/width < 1:3).

Moreover, the interconnection and said carrier substrate can be designed as one piece, preferably as a flexible printed circuit board with local stiff reinforcements. This facilitates the overall production process.

Furthermore, at least within a mid-section of the interconnection, a widths of the strain relief device may be smaller than or equal to a width of said interconnection. This feature is advantageous, because it avoids that the strain relief device, in particular in case it is formed as a flat metal ribbon, interacts with an outer silicone housing, which will be described in more detail below.

As has been explained at the beginning, the strain relief device can be bendable around a bending axis lying in said second plane. Even more preferably, the strain relief may also be twistable around a torsion axis perpendicular to said bending axis. These features allow easy use and applicability of the apparatus, in particular of the sensor head at the distal end, to the small heads of neonates.

Some details concerning preferred arrangements of the stress lines are important: For example, it has been explained already, that the two stress lines can be configured to transmit tensile stress from the electronic unit to the carrier substrate in a longitudinal axis. Furthermore, each of said tensile stress lines may run inside of or at borders of said interconnection and/or along or oblique to said longitudinal axis. This is in particular the case, if the strain relief device is formed as a flat ribbon of a width which is slightly smaller than a flat cable providing an electrical interconnection or a flat bundle of optical fibers providing an optical interconnection.

Furthermore, each of said tensile stress lines may start from an evaluation printed circuit board bearing said electronic unit and terminate on said carrier substrate.

In greater detail, the strain relief device may be configured to transmit tensile stress from a first point of attack at which a first of said two tensile stress lines is anchored to the electronic unit to a second point of attack at which a second of said two stress lines is anchored to said carrier substrate. In other words, the strain relief device may offer a third tensile stress line, connecting the first point of attack with the second point of attack. For this purpose and according to a preferred embodiment, the strain relief device may be formed as a ribbon.

Moreover, the strain relief device may be formed from a flexible, in particular metal, sheet. This sheet is preferably made from spring steel, because this material offers excellent flexibility and high strength, and most preferably with a thickness of less than 0.2 mm, which facilitates easy bending of the apparatus.

In another embodiment according to the disclosure the strain relief device features a Y-junction forming two distal arms, thereby offering a point of attack on each of the two arms for transmitting tensile stress to the carrier substrate (4), at the distal end of the apparatus. Each of these points of attack can transmit tensile stress to the carrier substrate. According to this specific design, the two points of attack on the two arms can be located at a distance from each other which is greater, preferably more than two times greater, than a lateral width of a mid-section of said interconnection. Thereby, the strain relief device can offer a large lever arm for taking up torque in the first plane. At the same time, the two points of attack on the two arms can be twisted around the longitudinal axis and/or shifted against each other out of the first plane. These features allows safe twisting and application of the sensor head of the apparatus.

Preferably, the two distinct points of attack can be located proximal with respect to and/or outside of a measurement region of said carrier substrate, in which the at least one light source and the at least one detector are arranged. This feature allows a high bendability of the carrier substrate, independently of the strain relief device, which is crucial for obtaining reliable optical measurements.

In accordance with a second aspect of the present disclosure, an apparatus is provided which achieves the objectives, introduced at the beginning. In particular, the disclosure proposes an apparatus as introduced at the beginning, which, in addition, is characterized in that a stiff reinforcement element, preferably designed as a plate, is arranged at said distal end as well as proximal to and remote from said carrier substrate, and wherein said reinforcement element mechanically supports said interconnection. It is noted here, that such an apparatus may have other features as previously described herein with respect to the first aspect of the present disclosure.

The advantage of placing such a reinforcement element proximal from and at a distance of the carrier substrate is that above the reinforcement element, the tracks of the interconnection may be routed obliquely to the longitudinal axis defined by the interconnection without risking an electrical or optical failure due to a crack. Thanks to the support provided by the reinforcement element, the tracks of the interconnection can safely fan-out in the area of the reinforcement element. Due to this feature, the tracks can be routed close to each other within a mid-section of the interconnection, resulting in an overall slim design of the apparatus. At the same time, with the tracks fanning out above the reinforcement element, the tracks can be guided to lateral points of the carrier substrate, that lie outside of a longitudinal projection of said mid-section, to provide electrical contacts or optical ports at these locations. Since the reinforcement element is also distant from the measuring printed board, the measuring printed board can maintain its flexibility, which is crucial for obtaining excellent skin contact during a measurement.

The reinforcement element may be fabricated along with said interconnection, in particular as an integral part of said interconnection, or separately from said interconnection, in particular and fixed, preferably glued, to said interconnection.

Particular of advantage for a high robustness of the apparatus is a design in which the reinforcement element is mechanically connected to the strain relief device, detailed above. This connection can be preferably made at two arms of the strain relief device.

As has been explained, above the reinforcement element the tracks may run obliquely with respect to the longitudinal axis, such that the tracks fan out. This fan out can be in particular such that a respective distance between a left outermost and a right outermost track of the tracks is larger above said reinforcement element as within a mid-section of the interconnection. Through the mid-section, the tracks may run in parallel. The tracks may also terminate in the measurement printed circuit board.

In accordance with a third aspect of the present disclosure, an apparatus is provided which achieves the objectives, introduced at the beginning. In particular, the invention proposes an apparatus as introduced at the beginning, which, in addition, is characterized in that said apparatus features a rupture detection device configured to electronically and/or optically detect the beginning of a rupture or crack in said interconnection and to, preferably optically and/or acoustically, issue a warning accordingly. It is noted here, that such an apparatus may have other features as previously described herein with respect to the first and/or second aspect of the present disclosure.

The primary advantage of such a rupture detection device is that a user can be warned, as soon as a rupture or crack begins to form in the interconnection in the area of a sensing track electronically and/or optically monitored by the rupture detection device. Accordingly, the user can try to fix the crack or - in the worst case - replace the interconnection or the whole apparatus and thus avoid a failure of the apparatus during operation.

To allow such monitoring, the rupture detection device may comprise a sensing track which runs, at least partly or fully, along a boundary line of the interconnection. Such a placement is ideal, since the mechanical stress reaches peak values at that boundary and hence, cracks and ruptures typically originate at the boundary. As a result, the very beginning of the rupture formation can be detected with the rupture detection device.

Most critical for rupture sensing is the mid-section between said electronic unit and said carrier substrate. Hence, the sensing track should run along said boundary at least in this section.

According to a preferred embodiment, the rupture detection device is configured to electronically detect a major change in an electrical resistivity of the sensing track. This approach is highly simple and yet reliable for electronically detecting the very beginning of a rupture.

Alternatively or additionally, the sensing track may be in part or fully formed by an optical fiber or the like. In this case, the rupture detection device may be configured to optically detect a major change in a transmission of light through the sensing track.

The sensing track can be formed from at least one additional conductive or optical track offered by the interconnection. Moreover, the sensing track may be electrically or optically connected to the electronic unit.

According to one embodiment, the interconnection may feature a mat composed of carbon-fibers running in the longitudinal direction. In this case one or more of said carbon-fibers, preferably located at the boundary line of the interconnection, may be employed as the sensing track of the rupture detection device, because such fibers show a sufficiently large conductivity in the direction of the fibers.

In a preferred design, the sensing track is electrically connected to the electronic unit in a voltage divider configuration. This allows detection of resistivity changes by a simple voltage or current measurement.

The sensing track may be formed in various ways. For example, the sensing track may be formed from a single track or it can be segmented into several tracks. Moreover, such tracks may be conductive or in the form of optical fibers.

In a specific design, the sensing track may start from the electronic unit and run along a first edge of the interconnection. In this case, it is preferably if the sensing track circumnavigates the carrier substrate, runs along a second edge of the interconnection, and finally terminates in the electronic unit. Alternatively, the sensing track may terminate in said carrier substrate, after running along said first edge. In all of these cases, it is possible and of advantage to conceive a design in which the sensing track encloses the tracks of the interconnection, at least in a mid-section, to allow sensing of ruptures occurring on any of the two edges of the interconnection.

Finally, to allow efficient protection and easy disinfection of the apparatus, the electronic unit, the interconnection, the carrier substrate, and the strain relief device may be embedded in a tube. This tube can be seamless and/or flexible; most preferably, it is made from a silicone.

According to an embodiment optimized for easy assembly, the tube features a distal opening through which the at least one wavelength can be emitted and received after being scattered by said medium and a proximal opening through which electrical signals are transferable by wire from the electronic unit to an external mobile device, for example a tablet. In this design, the tube has a shape which is tapered towards the distal end of the apparatus such that the assembled apparatus can be inserted through the distal opening into the tube and advanced inside the tube until said carrier substrate is located below said distal opening. Afterwards, the tube may be sealed, to protect the inner electronics of the apparatus.

Preferred embodiments of the present disclosure shall now be described in more detail, although the present invention is not limited to these embodiments.

With reference to the accompanying drawings, where features with corresponding technical function are referenced with same numerals even when these features differ in shape or design:
- Fig. 1: is an overall view of an apparatus, namely an NIRS-oximeter,
- Fig. 2: shows the oximeter of Fig.1 twisted around its longitudinal axis,
- Fig. 3: illustrates bending of the oximeter of Fig.1,
- Fig. 4: shows the inner components of the apparatus of Fig. 1 after removal of the silicone hull visible in detail in Fig. 9,
- Fig. 5: shows a top view on details of an electrical interconnection of the oximeter of Fig. 1,
- Fig. 6: shows a bottom view of the interconnection illustrated in Fig. 5,
- Fig. 7: shows an oblique side view on the top side of the interconnection illustrated in Fig. 5,
- Fig. 8: shows an oblique side view on the bottom side of the interconnection illustrated in Fig. 5,
- Fig. 9: shows details of the silicone hull visible also in Fig. 1-3,
- Fig. 10: illustrates the routing of conductive tracks of the interconnection of Fig. 5 - 8, and
- Fig. 11-15: illustrate different designs of an apparatus according to the invention, in which a carrier substrate of the apparatus carrying light sources and detectors is connected in different orientations to and electronic unit of the apparatus.

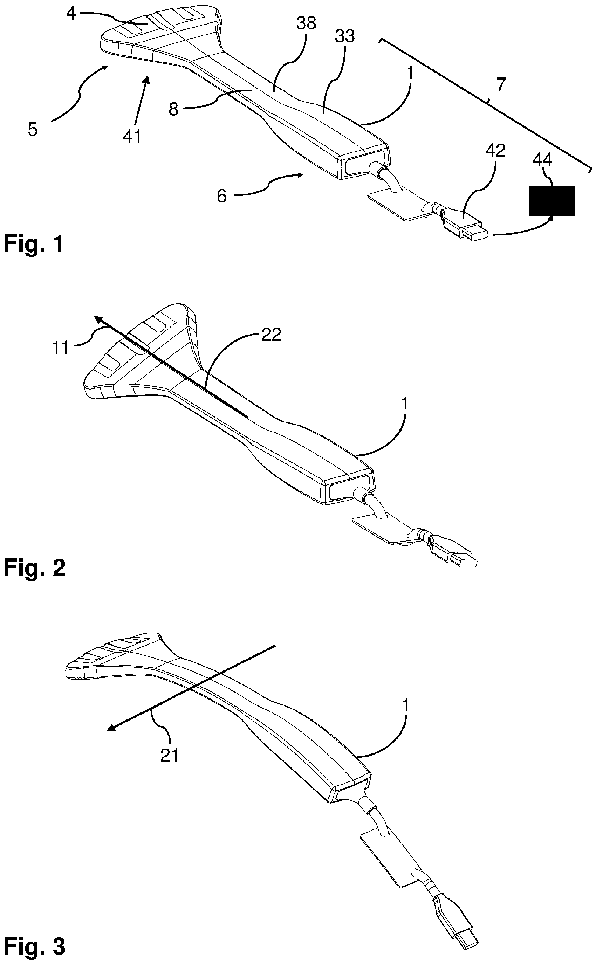

Figure 1 shows an apparatus 1, namely an oximeter designed for measuring cerebral oxygenation for vital-parameter-monitoring of infants. As is visible from figures 1 to 3, the apparatus 1 is elongated and highly flexible and can be bended and twisted, which is necessary to apply its sensor head 41 at its distal end 5 to the tiny heads of neonates.

Due to this bending, a high mechanical stress is applied, in particular to an electrical interconnection 8, in the form of a flat cable 9 with an aspect ratio of less than 1:5, connecting an electronic unit 7 located at a proximal end 6 of the apparatus 1 with a carrier substrate 4 located at a distal end 5 (c.f. Fig. 1) via several conductive tracks 10 (c.f.

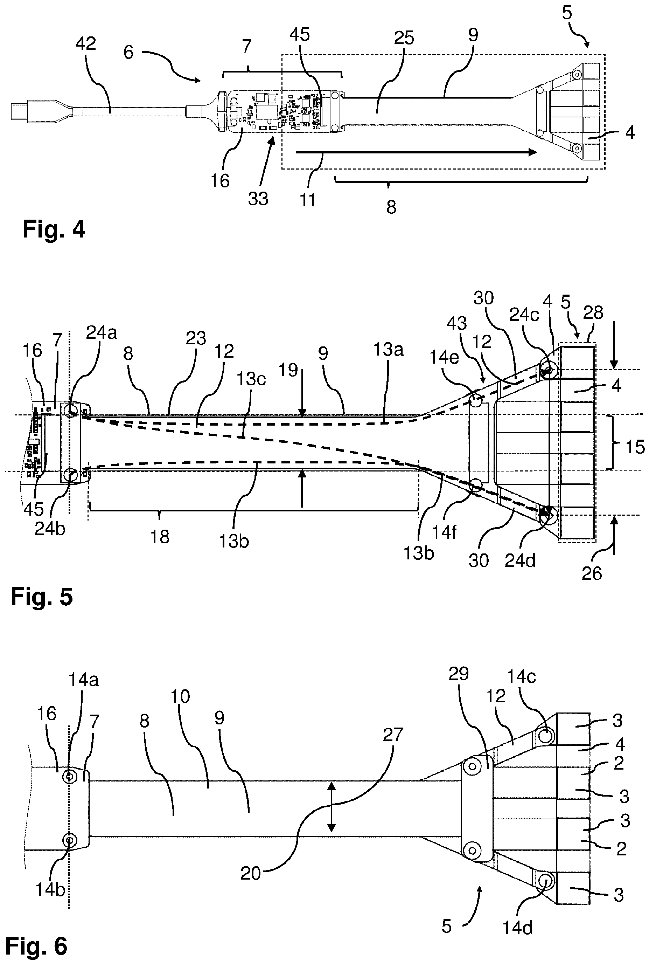

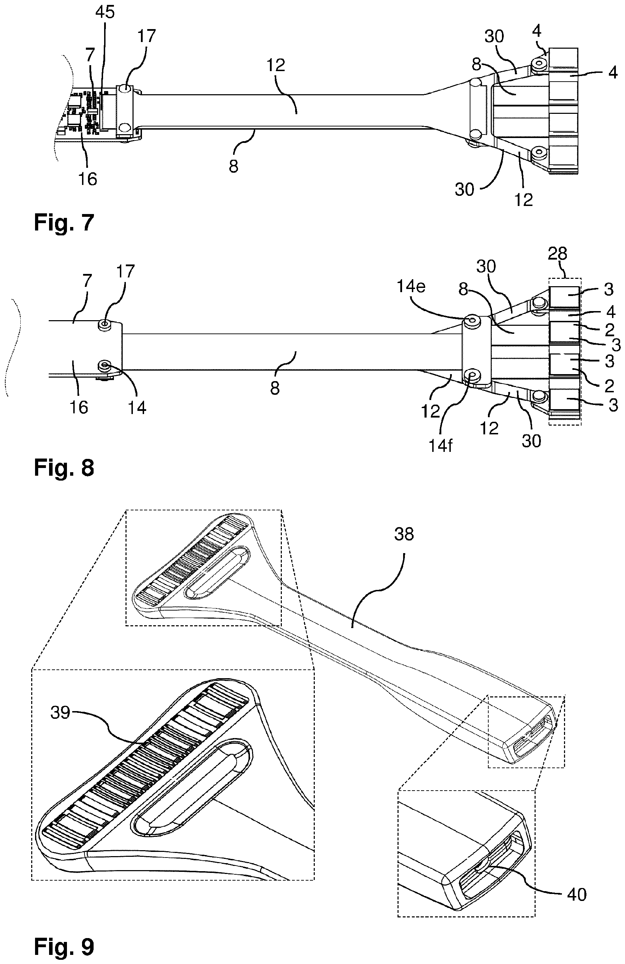

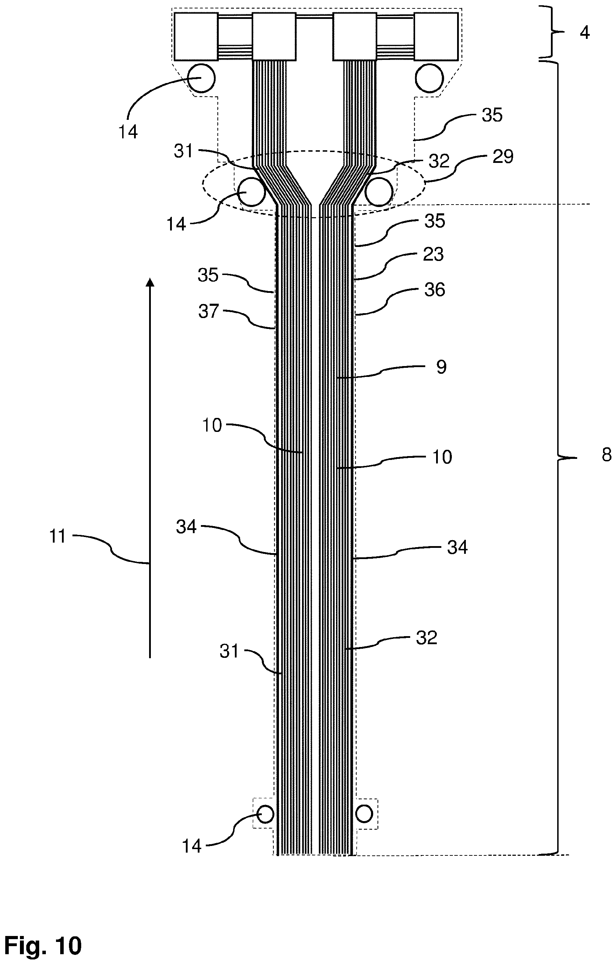

Fig. 10), as is best visible in Figure 4, which shows the inner components of the apparatus 1, after the outer silicone tube 38, shown in figure 9, has been removed.

As visible in figure 6, which shows the lower side of the carrier substrate 4, the latter bears several light sources 2 and several detectors 3, which are arranged such that several measurement wavelength can be emitted into the head of the neonate and detected by the detectors 3 after the light has been backscattered by the cerebral tissue inside the head. The electronic unit 7 is configured to control the light sources 2 and to evaluate electrical signals from the detectors 3. Based on these signals, the electronic unit 7 computes the oxygenation in the cerebral tissue and sends these data by a cable 42 (c.f. figure 1) to a tablet 44 (only indicated in figure 1), which forms part of the electronic unit 7, for visualizing these data.

As is visible in figure 10, the interconnection 8 features a number of conductive tracks 10 running in parallel within a mid-section 18 of the interconnection 8 along the longitudinal axis 11, also illustrated in figure 2. As is visible in figure 10, which shows a layout of the tracks 10, the tracks 10 define a first plane which comprises the longitudinal axis 11.

To avoid even tiny ruptures or cracks in the electrical interconnection 8, which can lead to a total failure of the apparatus 1 in the long term, the apparatus 1 features as strain relief device 12 (c.f. figures 4-8) configured to transmit tensile stress from the electronic unit 7 to the carrier substrate 4 along the longitudinal axis 11. For this purpose, the strain relief device 12 offers two tensile stress lines 13a, 13b, as illustrated in figure 5. A mechanical stress applied along one of these two lines 13a, 13b will be taken up by the strain relief device 12, which thus protects the electrical interconnection from excessive stress and resulting strain. Thereby, the danger of ruptures is greatly reduced.

Visible in Figure 5 is also an electrical interface 45 in the form of a plug-in connector, which connects the electrical interconnection 8 with the electronic unit 7. As indicated by the dotted vertical line in Figure 5, the strain relief device 12 is anchored in two proximal anchor points 14a, 14b (cf. Figure 6) to the electronic unit 7. Therefore, any stress applied between the distal end 5 of the apparatus 1 and the electronic unit 7 at the proximal end 6 of the apparatus 1 will be transmitted and taken up by the strain relief device 12, such that such stress cannot strain or even damage the electrical interconnection 8, in particular not at the location of the interface 45, which is located proximal to said proximal anchor points 14a, 14b.

The two tensile stress lines 13a, 13b define a second plane, which is oriented substantially parallel to the first plane defined by the conductive tracks 10 of the interconnection 8. As is visible from figures 2 and 3, not only the interconnection 8 but also the strain relief device 12 can be bent out of the first plane and twisted along the torsional axis 22 shown in figure 2. This high degree of flexibility of the strain relief device 12 is crucial to maintain the functionality of the oximeter.

As shown in figure 6, the two stress lines 13a, 13b and hence the strain relief device 12 are anchored to the electronic unit 7 by two proximal anchor points 14a, 14b and to the carrier substrate 4 by two distal anchor points 14c, 14d. A solid anchoring is achieved by rivets as connecting devices 17, which penetrate a stiff evaluation printed circuit board 16 of the electronic unit 7 and the carrier substrate 4, respectively. As indicated by thin dashed lines in figure 5, the anchor points 14a, 14b, 14c, and 14d lie outside of a longitudinal projection 15 of the conductive tracks 10 and are thus spaced by more than the width 20 of the mid-section of the interconnection 8.

From the location of the anchor points 14a, 14b, 14c, and 14d, it is also visible that the electronic unit 7 and the carrier substrate 4 are each wider in the first plane then the midsection 18 of the interconnection 8. In other words, the four anchor points 14a, 14b, 14c, and 14d lie outside of the longitudinal projection of the midsection 18. Due to the resulting large spacing between the two tensile stress lines 13a, 13b, the strain relief device 12 can efficiently take up a torque in the first plane of the interconnection 8 and thus also protect the interconnection against excessive shear forces.

Furthermore, it can be seen from Fig. 5 that each of the two stress lines 13a, 13b transmits tensile stress from the electronic unit 7 to the carrier substrate 4 along the longitudinal direction 11 (c.f. figure 4). This is because the stress lines start from the electronic unit 7 at the anchor points 14a, 14b and terminate on the carrier substrate 4 at the anchor points 14c, 14d. Also visible is that, due to the ribbon shape of the strain relief device 12, the latter transmits also stress from the point of attack 24a to the point of attack 24d (which correspond to the anchor points 14b and 14c, respectively) via a third tensile stress line 13c.

The strain relief device 12 is fabricated - separately from the interconnection 8 - from a 0.1 mm thin sheet of spring steel, which offers a product of cross-section A [mm2] times Young's modulus E [GPa] of larger than 5 kN and therefore a high rigidity.

As can be seen by comparison of the top view of figure 5 and the bottom view of figure 6, the width 19 of the strain relief device 12 is smaller than the width of the interconnection 8 in the mid-section 18. Therefore, the two stress lines 13a and 13b run inside of and partly along the left or right border 23 of said interconnection 8, respectively.

Furthermore, the strain relief device 12 offers two separate distal arms 30, thanks to the Y-junction 43 indicated in figure 5, and each of these two arms offers a point of attack 24c, 24d, respectively, for transmitting tensile stress to the carrier substrate 4. These points of attack are provided by the distal anchor points 14c and 14d, linking the strain relief device 12 to the carrier substrate 4. Points 24c and 24d show a distance from each other, which is more than 2.5 larger than the lateral width 27 of the mid-section 18, illustrated in figure 6. As can be imagined with a glimpse on figure 2 and 3, points 24c and 24d can be twisted around axis 11 and also shifted against each other out of the first plane when twisting the sensor head 41 of the apparatus 1.

As indicated by the dashed box in figure 5, points 24c and 24d are located proximal with respect to and outside of a measurement region 28 of said carrier substrate 4 in which the light sources 2 and detectors are arranged.

From the layout depicted in figure 10 it can be seen that the carrier substrate 4 and the interconnection 8 are fabricated as one piece of a flexible printed circuit board offering local stiff reinforcements.

At the distal end 5 of the apparatus 1, a stiff reinforcement element 29 in the form of a metal plate supports the electrical interconnection 8 in the area of the Y-junction (c.f. figure 6). As is indicated by numeral 29 in figure 10, above the plate, the tracks 10 fan-out such that a respective distance between a left outermost track 31 and a right outermost track 32 of said conductive tracks 10 is larger above said reinforcement element 29 as within the mid-section 18 of the interconnection 8, in which the tracks 10 run in parallel.

As the stiff reinforcement element 29 is positioned proximal to and remote from the carrier substrate 4, the latter can still be bent and twisted. At the same time, the support provided by element 29 protects the delicate tracks against ruptures. For this purpose, the stiff reinforcement element 29 is mechanically connected to each of the two arms 30 of the strain relief device 12 by rivets, as indicated by the anchor points 14e and 14f in figures 5 and 8.

Finally, there is a separate electric sensing track 34 discernible in figure 10 (thickest outermost of the conductive tracks 10), which forms the sensor element of a rupture detection device 33, which is implemented by the electronic unit 7 of the apparatus 1. The rupture detection device 33 is configured to detect even small changes of the resistivity of the electric sensing track 34, which will occur for example, if a rupture starts to form at the boundary line 35 of the interconnection 8. In such a case, the rupture detection device 33 issues an optical warning to a user by a LED, who can then inspect the apparatus 1.

As is visible from the layout in figure 10, the sensing track 34 is formed from two single conductive tracks 10 provided by the interconnection 8. In more detail, the sensing track 34 starts from the electronic unit 7 (which corresponds to the location of the proximal end of the interconnection 8 at the bottom of figure 10), runs along a first edge 36 of said interconnection 8 to the measurement printed circuit board 4, circumnavigates the measurement printed circuit board 4, and finally runs along a second edge 37 of the interconnection 8 back to the electronic unit 7. As is visible in figure 10, small stripes of PCB material separate the sensing track 34 from the outer edges 36 and 37, respectively.

Alternatively, the sensing track 34 running along the first edge 36 may terminate on a contact pad in the measurement printed circuit board 4 and this contact pad may be on a defined voltage level. In this case, the other branch of the sensing track 34 may likewise start from the electronic unit 7, run along the second edge 37 of the interconnection 8 and terminate in said measurement printed circuit board 4, in particular on a contact pad at the same voltage level, in particular said contact pad.

In both cases, the sensing track 34 will run fully along the boundary line 35 of the interconnection 8 in the mid-section 18. The sensing track 34 can thus sense those spots, where ruptures are most likely.

Figure 9 illustrates the silicone tube 38, which forms an outer hull of the apparatus 1, and which is also visible in figures 1-3 in greater detail. The complete assembly shown in figure 4 can be introduced with the electronic unit 7 first into the tube 38 through the distal opening 39 shown in figure 9. The assembly of figure 4 can then be advanced inside the tube 38 until the carrier substrate 4 is located below the distal opening 39 of the tube 38. The cable 42, linking electronic components of the electronic unit 7 to the tablet 44 can then be guided through the proximal opening 40, also displayed in figure 9, such that electrical signals are transferable by wire from proximal parts of the electronic unit 7 to the tablet 44.

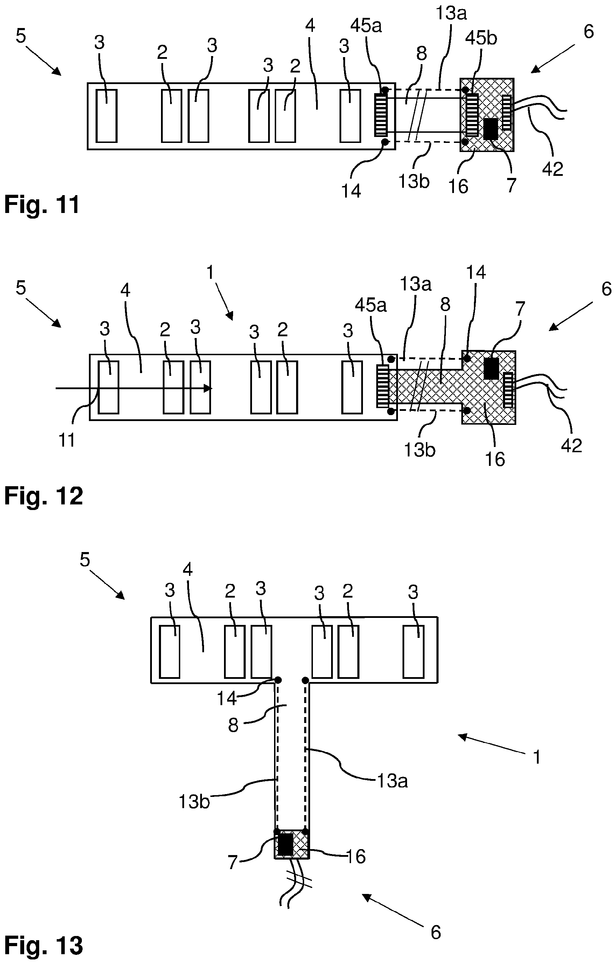

Finally figures 11 to 15 display (schematically) various possible designs of an apparatus according to the invention:
In the example of figure 11, other than in example of figure 10, the carrier substrate 4 and the interconnection 8 are realized as separate flexible printed circuit board (PCBs) (i.e. not as a one piece flex-PCB), which are electrically connected via a distal interface 45a. At the proximal end 6, the interconnection 8 is electrically connected via a proximal interface 45b to an evaluation PCB 16 bearing the electronic unit 7. Moreover, it is visible that the linear arrangement of light sources 2 and detectors 3 is now oriented along the longitudinal axis 11 of the apparatus and no longer perpendicular to axis 11, as in the previous example of figure 10.

In the example of figure 12, this longitudinal arrangement is maintained, however, the electrical interconnection 8 and the evaluation PCB 16 are formed as one piece in the form of a semi-flexible or flex only PCB (i.e. with or without local reinforcements), which is connected to the carrier substrate 4 via a distal interface 45a.

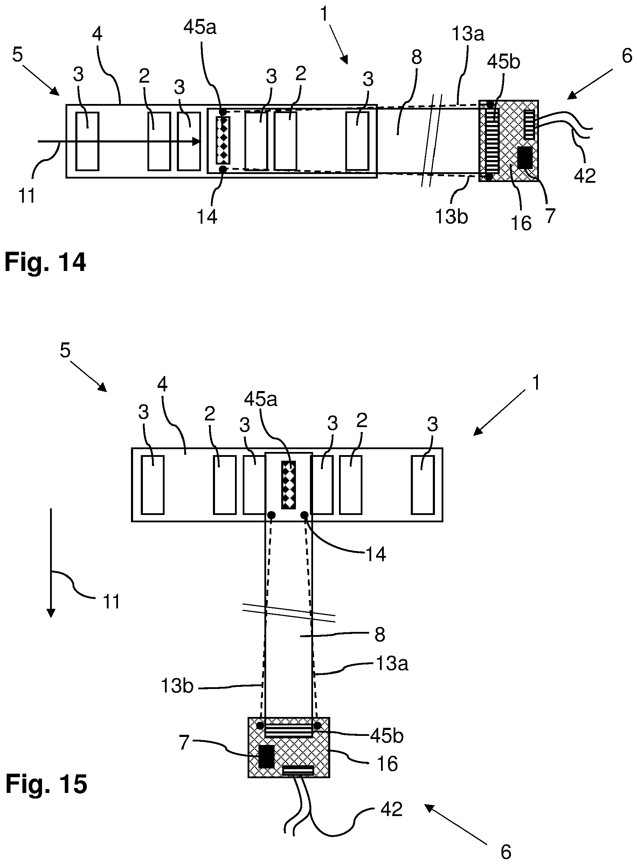

In figure 14, the longitudinal arrangement is again maintained, but the distal interface 45a has moved to a center position within the carrier substrate 4. The interconnection 8 is thus electrically connected to the center of the carrier substrate 4 at its distal end and to the evaluation PCB 16, realized as a separate PCB, via a proximal interface 45 b at its proximal end.

Figures 13 and 15 display two more designs following the T-shape of the example of figure 10, in which the linearly aligned light sources 2 and detectors 3 are oriented perpendicular to the longitudinal axis 11 of the apparatus 11 (resulting in an overall Hammer-shape of the apparatus 1 as exemplarily displayed in figure 1). In the example of figure 13, the carrier substrate 4, the electrical interconnection 8 and the evaluation PCB 16 bearing the electronic unit 7 are all fabricated as a single and T-shaped PCB which is flexible but shows local stiff reinforcement elements. Hence, no separate interfaces are necessary for routing signals from the control unit 7 to the light sources 2 and detectors 3.

Similar to the example of figure 12, in the designs of figures 14 and 15, the electrical interconnection 8 and the evaluation PCB 16 may also be designed as one piece (i.e. without using a proximal interface 45b).

In all of the above examples of figure 11 to 15, the dashed lines illustrate the two tensile stress lines 13a, 13b provided by a separate strain relief device 12 not illustrated in the schematics of figures 11 to 15. Each of these stress lines 13a, 13b can deliver stress from a proximal anchor point 14 to a distal anchor point 14 (the anchor points 14 are illustrated as black dots in the schematics) and thereby protect the delicate electrical tracks 10 of the interconnection 8 from ruptures, when stress is applied to the apparatus 1 along its longitudinal axis 11 during use.

In summary, several measures are suggested for improving the durability, lifetime and electrical reliability of an elongated and flexible apparatus 1 designed for measuring an optical or physiological parameter in a scattering medium, featuring an interconnection 8 linking an electronic unit 7 at a proximal end 6 of the apparatus 1 to a carrier substrate 4, bearing a light source 2 and detector 3, at a distal end 5 of the apparatus 1. These measures include a strain relief device 12 offering two separate stress lines 13a, 13b for transmitting tensile stress from a pair of spaced anchoring points 14a, 14b at the electronic unit 7 to a pair of spaced anchoring points 14c, 14d at the measuring circuit board, an electronic rupture detection device 33 configured to detect the formation of ruptures in the interconnection 8, and a stiff reinforcement element 29 located remote from and proximal to the measuring printed circuit 4 board for mechanically supporting the interconnection 8 in the area of a fan-out.

### List of reference numerals

- 1: apparatus
- 2: light source
- 3: detector
- 4: carrier substrate
- 5: distal end
- 6: proximal end
- 7: electronic unit
- 8: interconnection
- 9: flat cable
- 10: track
- 11: longitudinal axis
- 12: strain relief device
- 13: tensile stress line
- 14: anchor point
- 15: longitudinal projection (of 10)
- 16: evaluation printed circuit board
- 17: connecting device
- 18: mid-section (of 8)
- 19: width (of 12)
- 20: width (of 8)
- 21: bending axis
- 22: torsion axis
- 23: border (of 8)
- 24: point of attack
- 25: sheet
- 26: distance (between 24)
- 27: lateral width (of 18)
- 28: measurement region
- 29: reinforcement element
- 30: arm (of 12)
- 31: left outermost track
- 32: right outermost track
- 33: rupture detection device
- 34: sensing track
- 35: boundary line (of 8)
- 36: first edge (of 8)
- 37: second edge (of 8)
- 38: tube
- 39: distal opening
- 40: proximal opening
- 41: sensor head
- 42: cable
- 43: Y-junction
- 44: tablet
- 45: electrical interface

## Claims

1. **Elongated and bendable apparatus (1)** for measuring at least one optical or physiological parameter in a scattering medium, comprising
- at least one light source (2) and at least one detector (3) arranged on a carrier substrate (4) at a distal end (5) of said apparatus (1) such that
- said at least one detector (3) can detect at least one wavelength, emitted by said at least one light source (2) and scattered by said medium,
- an electronic unit (7) configured to control said at least one light source (2) and to evaluate electrical signals from said at least one detector (3) for computing said parameter, and located at a proximal end (6) of said apparatus (1), and
- an interconnection (8) connecting said electronic unit (7) with said carrier substrate (4) via several tracks (10) running in a longitudinal axis (11) of said apparatus (1),
- said apparatus (1) features a bendable strain relief device (12) configured to transmit tensile stress from said electronic unit (7) to said carrier substrate (4) along said longitudinal axis (11), and **characterised in that**
- said strain relief device (12) is provided in the form of two tension threads (13a, 13b), which are spaced from each other, and
- wherein said two tension threads (13a, 13b) are anchored to said electronic unit (7) by two proximal anchor points (14a, 14b) spaced from each other and to said carrier substrate (4) by two distal anchor points (14c, 14d) spaced from each other.

2. Apparatus (1) according to claim 1, wherein said strain relief device (12)
- forms an integral part of said interconnection (8) or
- has been fabricated separately from said interconnection (8)
and/or wherein the two tension threads (13a, 13b) each have the shape of a flat band or ribbon.

3. Apparatus (1) according to claim 1 or 2,
- wherein said proximal anchor points (14a, 14b) and/or said distal anchor points (14c, 14d) lie outside of a longitudinal projection (15) of said tracks (10),
- in particular wherein said carrier substrate (4) and said electronic unit (7) are each wider in said first plane than a mid-section (18) of said interconnection (8) and said anchor points (14a, 14b, 14c, 14d) each lie outside of a longitudinal projection of said mid-section (18) of said interconnection (8).

4. Apparatus (1) according to one of the preceding claims, wherein said strain relief device (12) features a cross-section A and is fabricated from a material with a Young's modulus E such that the product of A times E is greater than 2 kN,
- preferably wherein said Young's modulus E is greater than 20 GPa.

5. Apparatus (1) according to one of the preceding claims, wherein said interconnection (8) is designed as a flat cable (9), preferably with an aspect ratio smaller than 1:3, and/or
- wherein said interconnection (8) and said carrier substrate (4) are designed as one piece,
- preferably as a bendable printed circuit board with local stiff reinforcements, and/or
- wherein, at least within a mid-section (18) of said interconnection (8), a widths (19) of said strain relief device (12) is smaller than or equal to a width (20) of said interconnection (8).

6. Apparatus (1) according to one of the preceding claims,
- wherein said strain relief device (12) is bendable around a bending axis (21) lying in said second plane,
- preferably and twistable around a torsion axis (22) perpendicular to said bending axis (21).

7. Apparatus (1) according to one of the preceding claims,
- wherein each of said two tension threads (13a, 13b) is configured to transmit tensile stress from said electronic unit (7) to said carrier substrate (4) in said longitudinal axis (11) and/or
- wherein each of said tension threads (13a, 13b) runs inside of borders (23) of said interconnection (8) and/or along or oblique to said longitudinal axis (11) and/or
- wherein each of said tension threads (13a, 13b) starts from an evaluation printed circuit board (16) bearing said electronic unit (7) and terminates on said carrier substrate (4).

8. Apparatus (1) according to one of the preceding claims,
- wherein said strain relief device (12) is configured to transmit tensile stress from a first point of attack (24a) at which a first of said two tension threads (13a) is anchored to said electronic unit (7) to a second point of attack (24d) at which a second of said two tension threads (13b) is anchored to said carrier substrate (4).

9. Apparatus (1) according to one of the preceding claims,
- wherein the strain relief device (12) features a Y-junction forming two distal arms (30), thereby offering a point of attack (24c, 24d) on each of the two arms (30) for transmitting tensile stress to the carrier substrate (4),
- in particular wherein the two points of attack (24c, 24d) on the two arms (30) are located at a distance (26) from each other which is greater, preferably more than two times greater, than a lateral width (27) of a mid-section (18) of said interconnection (8) and/or
- can be twisted around said longitudinal axis and/or shifted against each other out of the first plane.

10. Apparatus (1) according to one of the preceding claims, **characterized in that**
- a stiff reinforcement element (29), preferably designed as a plate, is arranged at said distal end (5) as well as proximal to and remote from said carrier substrate (4), and
- said reinforcement element (29) mechanically supports said interconnection (8).

11. Apparatus (1) according to claim 10, wherein
- said reinforcement element (29) is mechanically connected to said strain relief device (12),
- preferably at two arms (30) of said strain relief device (12) .

12. Apparatus (1) according to claim 10 or 11,
- wherein above said reinforcement element (29) said tracks (10) run obliquely with respect to said longitudinal axis (11), such that said tracks (10) fan out,
- in particular such that a respective distance between a left outermost track (31) and a right outermost track (32) of said tracks (10) is larger above said reinforcement element (29) as within a mid-section (18) of said interconnection (8),
- in particular wherein said tracks (10) run in parallel through said mid-section (8) and/or terminate in said measurement printed circuit board (4).

13. Apparatus (1) according to one of the preceding claims,
- wherein said electronic unit (7), said interconnection (8), said carrier substrate (4), and said strain relief device (12) are embedded in a tube (38),
- preferably which is seamless and/or flexible and/or made from silicone,
- said tube (38) featuring a distal opening (39) through which said at least one wavelength can be emitted and received after being scattered by said medium and
- a proximal opening (40) through which electrical signals are transferable by wire from said electronic unit (7) to an external mobile device, for example a tablet (44), and
- wherein said tube (38) has a shape tapered towards said distal end (5) such that
- the assembled apparatus (1) can be inserted through the distal opening (39) into said tube (40) and advanced inside said tube (38) until said carrier substrate (4) is located below said distal opening (39).

## Patentansprüche

1. **Längliche und biegbare Vorrichtung (1)** zur Messung mindestens eines optischen oder physiologischen Parameters in einem streuenden Medium, umfassend
- mindestens eine Lichtquelle (2) und mindestens einen Detektor (3), der auf einem Trägersubstrat (4) an einem distalen Ende (5) der Vorrichtung (1) angeordnet ist, so dass
- der mindestens eine Detektor (3) mindestens eine Wellenlänge detektieren kann, die von der mindestens einen Lichtquelle (2) ausgestrahlt und von dem Medium gestreut wurde,
- eine Elektronikeinheit (7), die zum Steuern der mindestens einen Lichtquelle (2) und zum Evaluieren elektrischer Signale von dem mindestens einen Detektor (3) zur Berechnung des Parameters ausgelegt ist und die an einem proximalen Ende (6) der Vorrichtung (1) angeordnet ist, und
- eine Verbindung (8), die die Elektronikeinheit (7) über mehrere Bahnen (10), die in einer Längsachse (11) der Vorrichtung (1) verlaufen, mit dem Trägersubstrat (4) verbindet,
- wobei die Vorrichtung (1) eine biegbare Zugentlastungsvorrichtung (12) aufweist, die zum Übertragen von Zugspannung von der Elektronikeinheit (7) auf das Trägersubstrat (4) entlang der Längsachse (11) ausgelegt ist, **dadurch gekennzeichnet,**
- **dass** die Zugentlastungsvorrichtung (12) in Form von zwei voneinander beabstandeten Spannfäden (13a, 13b) bereitgestellt ist, und
- **dass** die zwei Spannfäden (13a, 13b) durch
- zwei proximale, voneinander beabstandete Verankerungspunkte (14a, 14b) an der Elektronikeinheit (7) und durch
- zwei distale, voneinander beabstandete Verankerungspunkte (14c, 14d) an dem Trägersubstrat (4)
verankert sind.

2. Vorrichtung (1) nach Anspruch 1, wobei die Zugentlastungsvorrichtung (12)
- einen integralen Bestandteil der Verbindung (8) bildet oder
- getrennt von der Verbindung (8) gefertigt wurde und/oder wobei die zwei Spannfäden (13a, 13b) jeweils die Form eines flachen Bands oder Streifens aufweisen.

3. Vorrichtung (1) nach Anspruch 1 oder 2,
- wobei die proximalen Verankerungspunkte (14a, 14b) und/oder die distalen Verankerungspunkte (14c, 14d) außerhalb eines Längsvorsprungs (15) der Bahnen (10) liegen,
- insbesondere wobei das Trägersubstrat (4) und die Elektronikeinheit (7) in der ersten Ebene jeweils breiter als ein mittlerer Abschnitt (18) der Verbindung (8) sind und die Verankerungspunkte (14a, 14b, 14c, 14d) jeweils außerhalb eines Längsvorsprungs des mittleren Abschnitts (18) der Verbindung (8) liegen.

4. Vorrichtung (1) nach einem der vorhergehenden Ansprüche, wobei die Zugentlastungsvorrichtung (12) einen Querschnitt A aufweist und aus einem Material mit einem Elastizitätsmodul E gefertigt ist, so dass das Produkt von A mal E größer als 2 kN ist,
- vorzugsweise wobei der Elastizitätsmodul E größer als 20 GPa ist.

5. Vorrichtung (1) nach einem der vorhergehenden Ansprüche, wobei die Verbindung (8) als ein flaches Kabel (9) ausgestaltet ist, vorzugsweise mit einem Aspektverhältnis von kleiner als 1:3, und/oder
- wobei die Verbindung (8) und das Trägersubstrat (4) einstückig ausgestaltet sind,
- vorzugsweise als eine biegbare Leiterplatte mit lokalen steifen Verstärkungen, und/oder
- wobei mindestens in einem mittleren Abschnitt (18) der Verbindung (8) eine Breite (19) der Zugentlastungsvorrichtung (12) gleich oder kleiner als eine Breite (20) der Verbindung (8) ist.

6. Vorrichtung (1) nach einem der vorhergehenden Ansprüche,
- wobei die Zugentlastungsvorrichtung (12) um eine in der zweiten Ebene liegende Biegeachse (21) biegbar ist,
- vorzugsweise und um eine Torsionsachse (22) senkrecht zur Biegeachse (21) verdrehbar ist.

7. Vorrichtung (1) nach einem der vorhergehenden Ansprüche,
- wobei die zwei Spannfäden (13a, 13b) jeweils zum Übertragen von Zugspannung von der Elektronikeinheit (7) auf das Trägersubstrat (4) in der Längsachse (11) ausgelegt sind, und/oder
- wobei die Spannfäden (13a, 13b) jeweils innerhalb von Grenzen (23) der Verbindung (8) und/oder entlang oder schräg zu der Längsachse (11) verlaufen, und/oder
- wobei die Spannfäden (13a, 13b) jeweils an einer die Elektronikeinheit (7) tragenden Evaluierungsleiterplatte (16) beginnen und an dem Trägersubstrat (4) enden.

8. Vorrichtung (1) nach einem der vorhergehenden Ansprüche,
- wobei die Zugentlastungsvorrichtung (12) zum Übertragen von Zugspannung von einem ersten Angriffspunkt (24a), an dem ein erster der zwei Spannfäden (13a) an der Elektronikeinheit (7) verankert ist, auf einen zweiten Angriffspunkt (24d), an dem ein zweiter der zwei Spannfäden (13b) am Trägersubstrat (4) verankert ist, ausgelegt ist.

9. Vorrichtung (1) nach einem der vorhergehenden Ansprüche,
- wobei die Zugentlastungsvorrichtung (12) eine Y-Verzweigung aufweist, die zwei distale Arme (30) bildet, wodurch ein Angriffspunkt (24c, 24d) an jedem der zwei Arme (30) zur Übertragung von Zugspannung auf das Trägersubstrat (4) bereitgestellt wird,
- insbesondere wobei die zwei Angriffspunkte (24c, 24d) an den beiden Armen (30) in einem Abstand (26) voneinander angeordnet sind, der größer, vorzugsweise mehr als das Doppelte größer, als eine seitliche Breite (27) eines mittleren Abschnitts (18) der Verbindung (8) ist, und/oder
- um die Längsachse verdreht und/oder gegeneinander aus der ersten Ebene verschoben werden können.

10. Vorrichtung (1) nach einem der vorhergehenden Ansprüche, wobei
- ein steifes Verstärkungselement (29), vorzugsweise welches als eine Platte ausgelegt ist, an dem distalen Ende (5) und proximal zu und entfernt von dem Trägersubstrat (4) angeordnet ist, und
- wobei das Verstärkungselement (29) die Verbindung (8) mechanisch unterstützt.

11. Vorrichtung (1) nach Anspruch 10, wobei
- das Verstärkungselement (29) mechanisch mit der Zugentlastungsvorrichtung (12) verbunden ist,
- vorzugsweise an zwei Armen (30) der Zugentlastungsvorrichtung (12).

12. Vorrichtung (1) nach Anspruch 10 oder 11,
- wobei über dem Verstärkungselement (29) die Bahnen (10) schräg bezüglich der Längsachse (11) verlaufen, so dass die Bahnen (10) sich auffächern,
- insbesondere so, dass ein jeweiliger Abstand zwischen einer linken äußersten Bahn (31) und einer rechten äußersten Bahn (32) der Bahnen (10) über dem Verstärkungselement (29) größer als in einem mittleren Abschnitt (18) der Verbindung (8) ist,
- insbesondere wobei die Bahnen (10) parallel durch den mittleren Abschnitt (8) verlaufen und/oder in der Messungsleiterplatte (4) enden.

13. Vorrichtung (1) nach einem der vorhergehenden Ansprüche,
- wobei die Elektronikeinheit (7), die Verbindung (8), das Trägersubstrat (4) und die Zugentlastungsvorrichtung (12) in einem Rohr (38) eingebettet sind,
- vorzugsweise welches Rohr (38) nahtlos und/oder flexibel und/oder aus Silikon gefertigt ist,
- wobei das Rohr (38) eine distale Öffnung (39) aufweist, durch die die mindestens eine Wellenlänge ausgestrahlt und nach dem Streuen durch das Medium empfangen werden kann, und
- eine proximale Öffnung (40), durch die elektrische Signale über Kabel von der Elektronikeinheit (7) zu einem externen Mobilgerät, beispielsweise einem Tablet (44) übertragbar sind, und
- wobei das Rohr (38) eine Form aufweist, die sich zum distalen Ende (5) hin verjüngt, so dass
- die montierte Vorrichtung (1) durch die distale Öffnung (39) in das Rohr (40) eingeführt und innerhalb des Rohrs (38) vorgeschoben werden kann, bis das Trägersubstrat (4) unter der distalen Öffnung (39) angeordnet ist.

## Revendications

1. Appareil allongé et pliant (1) pour mesurer au moins un paramètre optique ou physiologique dans un milieu de diffusion, comprenant
- au moins une source de lumière (2) et au moins un détecteur (3) agencés sur un substrat de support (4) au niveau d'une extrémité distale (5) dudit appareil (1) de telle sorte que
- ledit au moins un détecteur (3) peut détecter au moins une longueur d'onde, émise par ladite au moins une source de lumière (2) et diffusée par ledit milieu,
- une unité électronique (7) configurée pour commander ladite au moins une source de lumière (2) et pour évaluer des signaux électriques provenant dudit au moins un détecteur (3) pour calculer ledit paramètre, et située à une extrémité proximale (6) dudit appareil (1), et
- une interconnexion (8) reliant ladite unité électronique (7) audit substrat de support (4) via plusieurs pistes (10) s'étendant selon un axe longitudinal (11) dudit appareil (1),
- ledit appareil (1) présente un dispositif antitraction pliant (12) configuré pour transmettre une contrainte de traction de ladite unité électronique (7) audit substrat de support (4) le long dudit axe longitudinal (11), et **caractérisé en ce que**
- ledit dispositif antitraction (12) se présente sous la forme de deux lignes de contrainte (13a, 13b), qui sont espacées l'une de l'autre, et
- dans lequel lesdits deux lignes de contrainte (13a, 13b) sont ancrées à ladite unité électronique (7) par deux points d'ancrage proximaux (14a, 14b) espacés l'un de l'autre et audit substrat de support (4) par deux points d'ancrage distaux (14c, 14d) espacés l'un de l'autre.

2. Appareil (1) selon la revendication 1, dans lequel ledit dispositif antitraction (12)
- fait partie intégrante de ladite interconnexion (8) ou
- a été fabriqué séparément de ladite interconnexion (8)
et/ou dans lequel les deux lignes de contrainte (13a, 13b) présentent chacune la forme d'une bande ou d'un ruban plat.

3. Appareil (1) selon la revendication 1 ou 2,
- dans lequel lesdits points d'ancrage proximaux (14a, 14b) et/ou lesdits points d'ancrage distaux (14c, 14d) se trouvent à l'extérieur d'une saillie longitudinale (15) desdites pistes (10),
- en particulier, dans lequel ledit substrat de support (4) et ladite unité électronique (7) sont chacun plus larges dans ledit premier plan qu'une section médiane (18) de ladite interconnexion (8) et lesdits points d'ancrage (14a, 14b, 14c, 14d) se trouvent chacun à l'extérieur d'une projection longitudinale de ladite section médiane (18) de ladite interconnexion (8).

4. Appareil (1) selon l'une quelconque des revendications précédentes, dans lequel ledit dispositif antitraction (12) présente une section transversale A et est fabriqué à partir d'un matériau avec un module de Young E de telle sorte que le produit de A par E soit supérieur à 2 kN,
- de préférence dans lequel ledit module de Young E est supérieur à 20 GPa.

5. Appareil (1) selon l'une quelconque des revendications précédentes, dans lequel ladite interconnexion (8) est conçue sous la forme d'un câble plat (9), de préférence avec un rapport d'aspect inférieur à 1:3, et/ou
- dans lequel ladite interconnexion (8) et ledit substrat de support (4) sont conçus d'un seul tenant,
- de préférence sous la forme d'une carte de circuit imprimé pliante avec des renforts rigides locaux, et/ou
- dans lequel, au moins à l'intérieur d'une section médiane (18) de ladite interconnexion (8), une largeur (19) dudit dispositif antitraction (12) est inférieure ou égale à une largeur (20) de ladite interconnexion (8).

6. Appareil (1) selon l'une quelconque des revendications précédentes,
- dans lequel ledit dispositif antitraction (12) peut être plié autour d'un axe de pliage (21) situé dans ledit second plan,
- de préférence et pouvant être tordu autour d'un axe de torsion (22) perpendiculaire audit axe de pliage (21).

7. Appareil (1) selon l'une quelconque des revendications précédentes,
- dans lequel chacun desdits deux lignes de contrainte (13a, 13b) est configuré pour transmettre une contrainte de traction de ladite unité électronique (7) audit substrat de support (4) selon ledit axe longitudinal (11) et/ou
- dans lequel chacun desdits fils de tension (13a, 13b) s'étend à l'intérieur de bordures (23) de ladite interconnexion (8) et/ou le long ou obliquement par rapport audit axe longitudinal (11) et/ou
- dans lequel chacun desdites lignes de contrainte (13a, 13b) part d'une carte de circuit imprimé d'évaluation (16) portant ladite unité électronique (7) et se termine sur ledit substrat de support (4).

8. Appareil (1) selon l'une quelconque des revendications précédentes,
- dans lequel ledit dispositif antitraction (12) est configuré pour transmettre une contrainte de traction à partir d'un premier point d'attaque (24a) au niveau duquel une première desdites deux lignes de contrainte (13a) est ancrée à ladite unité électronique (7) à un second point d'attaque (24d) au niveau duquel une seconde desdites deux lignes de contrainte (13b) est ancrée audit substrat de support (4).

9. Appareil (1) selon l'une quelconque des revendications précédentes,
- dans lequel le dispositif antitraction (12) présente une jonction en Y formant deux bras distaux (30), en offrant ainsi un point d'attaque (24c, 24d) sur chacun des deux bras (30) pour transmettre une contrainte de traction au substrat de support (4),
- en particulier dans lequel les deux points d'attaque (24c, 24d) sur les deux bras (30) sont situés à une distance (26) l'un de l'autre qui est supérieure, de préférence plus de deux fois supérieure, à une largeur latérale (27) d'une section médiane (18) de ladite interconnexion (8) et/ou
- peuvent être torsadés autour dudit axe longitudinal et/ou décalés l'un par rapport à l'autre hors du premier plan.

10. Appareil (1) selon l'une quelconque des revendications précédentes, **caractérisé en ce que**
- un élément de renfort rigide (29), de préférence conçu sous la forme d'une plaque, est agencé au niveau de ladite extrémité distale (5) ainsi qu'à proximité et à distance dudit substrat de support (4), et
- ledit élément de renfort (29) supporte mécaniquement ladite interconnexion (8).

11. Appareil (1) selon la revendication 10, dans lequel
- ledit élément de renfort (29) est relié mécaniquement audit dispositif antitraction (12),
- de préférence au niveau de deux bras (30) dudit dispositif antitraction (12).

12. Appareil (1) selon la revendication 10 ou 11,
- dans lequel, au-dessus dudit élément de renfort (29), lesdites pistes (10) s'étendent obliquement par rapport audit axe longitudinal (11), de telle sorte que lesdites pistes (10) s'étendent en éventail,
- en particulier de telle sorte qu'une distance respective entre une piste la plus extérieure gauche (31) et une la plus extérieure droite (32) desdites pistes (10) soit plus grande au-dessus dudit élément de renfort (29) qu'à l'intérieur d'une section médiane (18) de ladite interconnexion (8),
- en particulier dans lequel lesdites pistes (10) s'étendent en parallèle à travers ladite section médiane (8) et/ou se terminent dans ladite carte de circuit imprimé de mesure (4).

13. Appareil (1) selon l'une quelconque des revendications précédentes,
- dans lequel ladite unité électronique (7), ladite interconnexion (8), ledit substrat de support (4) et ledit dispositif antitraction (12) sont intégrés dans un tube (38),
- de préférence sans soudure et/ou flexible et/ou réalisé en silicone,
- ledit tube (38) présentant une ouverture distale (39) à travers laquelle ladite au moins une longueur d'onde peut être émise et reçue après avoir été diffusée par ledit milieu, et
- une ouverture proximale (40) à travers laquelle des signaux électriques peuvent être transférés par fil de ladite unité électronique (7) à un dispositif mobile externe, par exemple une tablette (44), et
- dans lequel ledit tube (38) présente une forme effilée vers ladite extrémité distale (5) de telle sorte que
- l'appareil assemblé (1) peut être inséré à travers l'ouverture distale (39) jusque dans ledit tube (40) et avancé à l'intérieur dudit tube (38) jusqu'à ce que ledit substrat de support (4) soit situé en dessous de ladite ouverture distale (39).
